# EUROPEAN PATENT SPECIFICATION

(11) **EP 1 498 732 B1**
(45) Date of publication and mention of the grant of the patent: **24.12.2014**
(21) Application number: 04254307.4
(22) Date of filing: 16.07.2004
(51) Int. Cl.: G01N 33/543, G01N 33/92, B01D 39/00

(54) **A one-step assay for high-density lipoprotein cholesterol**
Einschritt-Test für HDL-Cholesterin
Essai en une êtape du cholestêrol HDL

(30) Priority: 17.07.2003 US 488027 P
(43) Date of publication of application: 19.01.2005
(73) Proprietor: ORTHO-CLINICAL DIAGNOSTICS, INC., Rochester, NY 14626-5101 (US)
(72) Inventor: DiMagno, Theodore John, Penfield, New York 14526 (US); Arter, Thomas Charles, Rochester, New York 14612 (US)
(74) Representative: Bullett, Rachel Margaret

(56) References cited:
- EP-A- 1 342 792
- WO-A-02/38800
- WO-A-03/056163
- US-A- 4 892 815
- ARTER T ET AL: "Evaluation of the VITROS 5,1 FS Chemistry System Direct HDL Cholesterol MicroTipTM Assay" CLINICAL CHEMISTRY, vol. 48, no. 6 Supplement, June 2002 (2002-06), page A108, XP001204164 & 54TH ANNUAL MEETING OF THE AMERICAN ASSOCIATION FOR CLINICAL CHEMISTRY (AACC); ORLANDO, FLORIDA, USA; JULY 28-AUGUST 01, 2002 ISSN: 0009-9147
- PATENT ABSTRACTS OF JAPAN vol. 017, no. 412 (P-1583), 30 July 1993 (1993-07-30) & JP 05 080056 A (KONICA CORP), 30 March 1993 (1993-03-30)

## Description

### Background

A lipoprotein can be categorized as a high-density lipoprotein (HDL), a low-density lipoprotein (LDL), a very low-density lipoprotein (VLDL), or a chylomicron (CM). It is well known that HDL serves a protective function by removing cholesterol accumulated in tissues, including the arterial walls, and then returning it to the liver. Therefore, the cholesterol in HDL, also known as high-density lipoprotein cholesterol (HDLC), is a negative risk factor for various types of arteriosclerosis, such as coronary arteriosclerosis, and the HDLC level in blood is a useful index for the precognition of arteriosclerosis.

Solution assays are conventional methods used for determining the amount of HDLC in the blood. These methods consist of two steps, a fractionation step and a detection step. The fractionation step separates HDL from other lipoproteins whereas the detection step quantifies the cholesterol in the HDL. Examples of fractionation methods include an ultracentrifugation method, an immunochemical method, an electrophoretic method, and a precipitation method. An alternative approach to conventional solution methods is to perform the HDLC assay in a single step dry slide analytical element, also known as a dry slide, on a polyester support.

The development of the direct HDLC dry slide requires a preferential selectivity for HDLC over low-density lipoprotein cholesterol (LDLC), very low-density lipoprotein cholesterol (VLDLC), and cholesterol in CM. Many well-known solution methods have been shown to improve HDLC specificity by including non-high density lipoprotein precipitation methods (1, 2, 3, 4, 5), immune-inhibition (6,7), selective surfactants (8, 9, 10, 11), Catalase elimination (12), and polyethylene glycol (PEG) cross-linked cholesterol esterase (13). However, the precipitation methods and selective surfactant methods cannot be done as single-step dry slide assays because they do not yield the sufficient HDLC selectivity needed for such a method. Other methods use multiple reagent additions or separation steps that also are not amenable to the all inclusive single-step dry slide technology. Because none of the well-known HDLC methods yield sufficient selectivity in the dry slide assay, additional methods to improve HDLC specificity were pursued.

Surfactants are surface active agents that can alter the properties of fluid interfaces between polar and non-polar moieties, It has been well known among persons skilled in the art that surfactants can be used to selectively disrupt protein membranes or selectively solubilize their components. Surfactant solubilization methods have been used for several decades to purify proteins from a wide class of human, animal, and bacterial sources (14, 15, 16, 17). A surfactant's hydrophile-lipophile balance (HLB) number indicates the relative strength of the hydrophilic and hydrophobic areas of the surfactant molecule and characterizes the surfactant's relative affinity for aqueous (polar) and organic phases (non-polar). For instance, it is well documented that polyethylene oxide chain surfactants with HLB numbers ranging from 12.5 to 13.5 are effective in selectively solubilizing high-density lipoproteins in solution (18, 19). In addition, non-ionic surfactants with LB values less than 14.6 (20) have been found to preferentially solubilize LDL in solution. These general surfactant properties have been used in the development of several HDLC solution assays for automated analyzers (10, 11). For example, Matsui *et. al*. use non-ionic polyalkylene oxide surfactants with HLB numbers of 13 to 14 (lines 2-4 on page 4, ref 10) to selectively solubilize LDL in their assay. After LDL is solubilized, Matsui et al. use Catalase to eliminate the LDLC-derived peroxide produced. The addition of the second reagent then inhibits the Catalase in the first reaction cascade and begins a second reaction sequence by solubilizing the remaining HDL. Use of this two-step elimination method in Matsui et al. is not possible in the single-step dry slide assay (Scheme 1). In addition, the surfactant concentration range claimed by Matsui *et al*. (0.05 - 3%, lines 14-15 on page 4, ref 10) is insufficient to selectively solubilize HDL in the approximate 5.5 minute time frame amenable to the dry slide format.

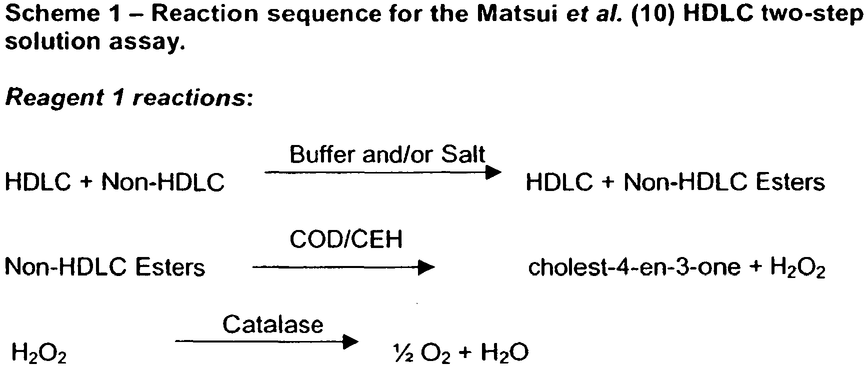

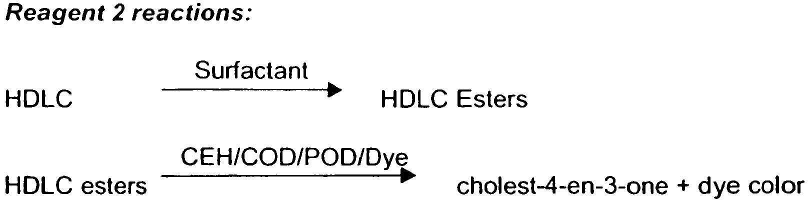

Hino et al. also disclose the use of non-ionic polyalkylene oxide surfactants with a HLB number of about 13. Like Matsui et al., Hino et al.'s use of surfactants is also not compatible with a dry slide assay (11). Hino et al. use a low concentration of surfactant in the range of 0.01 to 1% by weight that preferably does not dissolve lipoproteins. Along with the surfactant, a reagent is added which forms a complex with the non-high density lipoproteins. The complexed non-high density lipoproteins are then prevented from reacting with the enzymes used in the detection step of the assay. The enzymes used in the detection step are then added in the second reagent, which also contains the surfactant TRITON X-100 (a detergent produced by Dow Chemical) (lines 60-64 in column 2, ref 11). TRITON X-100 (TX-100) presumably solubilizes the uncomplexed HDL so that HDLC can react with the cholesterol detecting enzyme cascade (Scheme 2). Thus, the HDL selective surfactant in this solution assay has an inhibitory effect on the interaction between the detection enzymes and non-HDLs. The structure of the dry slide is not compatible with the above-described two-reagent addition method since all the reagents are contained together in the dry slide element and the TX-100 resolubilizes the precipitated non-HDL complexes in the dry slide format.

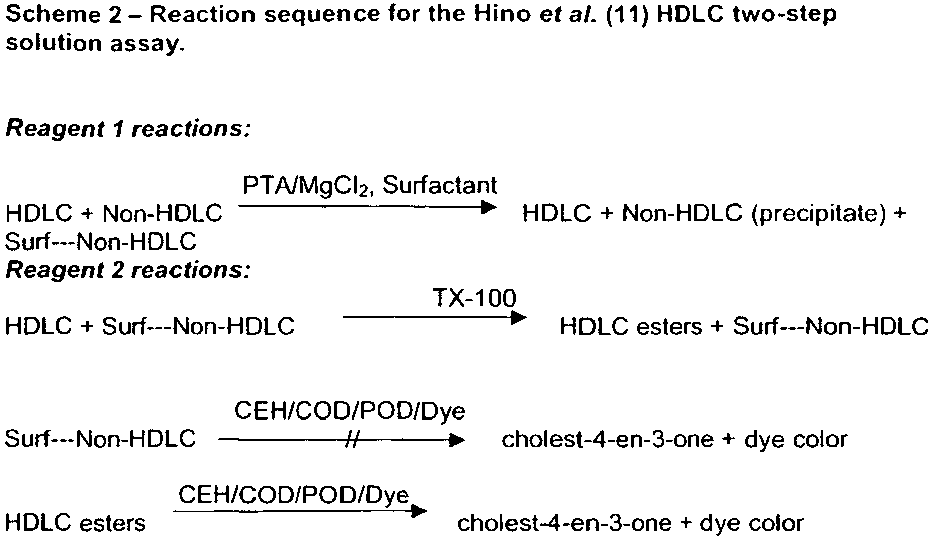

Since neither of the reaction mechanisms nor multiple reagent additions described in the above references are amenable to the all inclusive dry slide, new methods to employ the general property of selective surfactants need to be developed for the direct HDLC assay in a dry slide.

The effect of various surfactants on HDLC selectivity of the dry slide was assessed in combination with the other known effectors of HDLC selectivity. Reagents coated with selective surfactant in the dry slide to improve the HDLC selectivity include combinations of different non-HDL precipitating reagents (e.g., phosphotungstic acid, dextran sulfate, polyethylene glycol), ion exchange resins, LDL complex formers (calix[8]arene (21)), magnetic particles (22), and HDL selective cholesterol esterase (CEH) enzyme sources.

The present inventors have found two surfactants useful in conferring HDLC selectivity in a single-step HDLC dry slide assay. These surfactants confer HDLC selectivity in the reaction of cholesterol esterase with lipoproteins. The HDL selectivity observed with these surfactants in the direct HDLC dry slide is unusual compared to the lack of selectivity shown by other screened surfactant sources because the two surfactants disclosed retain their selectivity in the presence of polyanion-non-HDL-lipoprotein complexes. These surfactants with HDL selectivity, when used in conjunction with polyanion precipitation and an HDL selective cholesterol esterase, adds a third selectivity mechanism to the assay. The additional selectivity from these surfactants greatly improves the overall selectivity of the assay and makes it possible to have a functional single-step direct HDLC dry slide assay. Without this enhancement, the overall selectivity and accuracy of the assay is insufficient due to the interference from non-high density lipoprotein cholesterol.

### Summary of the invention

An object of the present invention is a method to provide for quantifying cholesterol in high-density lipoproteins using a single-step assay. A multi-layer analytical element is used wherein at least one layer contains phosphotungstic acid and another contains a surfactant that selectively acts on high-density lipoproteins and does not solubilize polyanion-non-high density lipoprotein complexes. In the present invention, the multi-layer analytical element is contacted with a sample that may contain high-density lipoprotein cholesterol. The non-HDL is precipitated and the HDL is solubilized in the spreading layer. Then the CEH reacts with the solubilized HDL cholesterol esters to form cholesterol. Finally the cholesterol in the high-density lipoprotein is detected and quantified.

In another embodiment, the surfactant is selected from EMULGEN B-66, EMULGEN A-90, or a mixture thereof.

In another embodiment, the amount of phosphotungstic acid present in a layer of the dry slide is preferably in an amount of 1 to 5 g/m².

In another embodiment, the amount of surfactant present in a layer of the dry slide is preferably in an amount of 3 to 8 g/m².

### Brief Description of the Drawings

Figure 1 shows a kinetic response for human HDL and LDL containing test fluids with the non-specific EMULGEN 109P surfactant without the presence of phosphotungstic acid.
Figure 2 shows a kinetic response for human HDL and LDL containing test fluids with the non-specific EMULGEN 109P surfactant in the presence of phosphotungstic acid.
Figure 3 shows a kinetic response for human HDL and LDL containing test fluids with HDL specific EMULGEN B-66 without the presence of phosphotungstic acid.
Figure 4 shows a kinetic response for human HDL and LDL containing test fluids with HDL specific EMULGEN B-66 in the presence of phosphotungstic acid.
Figure 5 shows a patient accuracy plot with the use of the non-selective surfactant EMULGEN 109P in the direct HDLC dry slide.
Figure 6 shows a patient accuracy plot with the use of the non-selective surfactant EMULGEN 220 in the direct HDLC dry slide.
Figure 7 shows a patient accuracy plot with the use of the HDL selective surfactant EMULGEN B-66 in the direct HDLC dry slide.
Figure 8 shows a patient accuracy plot with the use of the HDL selective surfactant EMULGEN A-90 in the direct HDLC dry slide.
Figure 9 shows a patient accuracy plot with the use of the non-selective surfactant EMULGEN 109P in the presence of phosphotungstic acid in the direct HDLC dry slide.
Figure 10 shows a patient accuracy plot with the use of the non-selective surfactant EMULGEN 220 in the presence of phosphotungstic acid in the direct HDLC dry slide.
Figure 11 shows a patient accuracy plot with the use of the HDL selective surfactant EMULGEN B-66 in the presence of phosphotungstic acid in the direct HDLC dry slide.
Figure 12 shows a patient accuracy plot with the use of the HDL selective surfactant EMULGEN A-90 in the presence of phosphotungstic acid in the direct HDLC dry slide.
Figure 13 shows a kinetic response for non-HDL precipitated with phosphotungstic acid and MgCl₂, resuspended in NaCl, TRITON X-100, and EMULGEN B-66, and tested on a coating containing EMULGEN B-66 surfactant.

### Detailed Description of the Invention

In the direct HDLC dry slide, two HDL selective surfactants were discovered which showed superior selectivity for HDL. Several surfactants were screened for HDLC selectivity (Table 1), but only two surfactants, EMULGEN B-66 (a polyoxyethylene derivative produced by KAO Corp.,) and EMULGEN A-90 (a polyoxyethylene derivative produced by KAO Corp.,) possessed the level of HDLC selectivity needed for a single-step direct HDLC dry slide assay. The superior HDL specificity of EMULGEN B-66 and EMULGEN A-90 is crucial to the direct HDLC dry slide because the currently known HDL specific methods do not provide adequate HDLC specificity needed for the dry slide assay. One example of the dry slide used in evaluating the various surfactants is shown in example 1, however, the location of the enzymes and other reactive ingredients may be placed in a variety of positions within the dry slide and a variety of different materials may be used for the various layers.

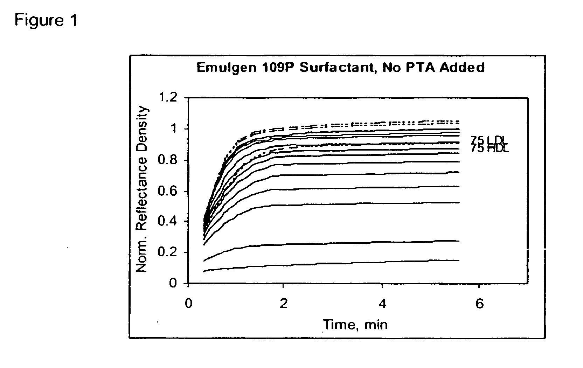

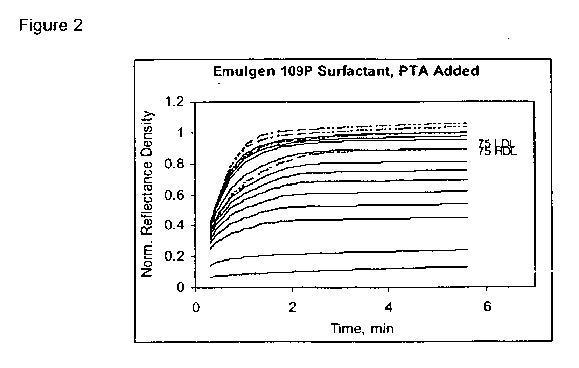

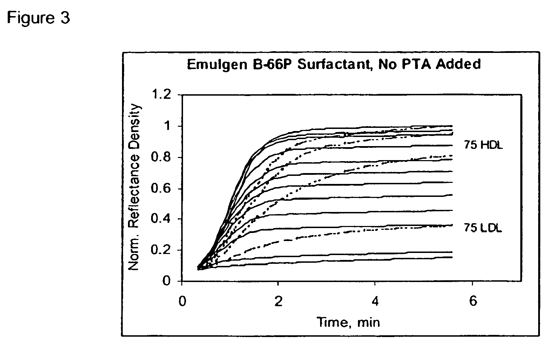

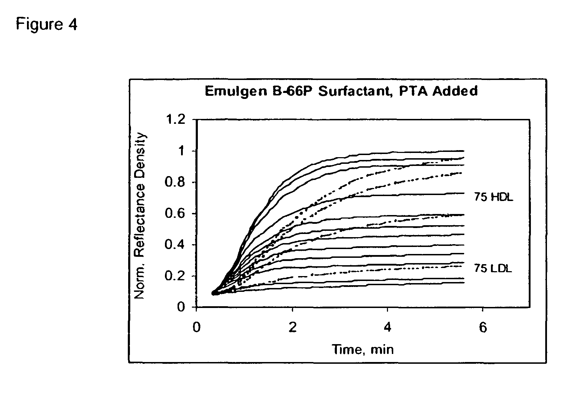

The HDL selectivity of EMULGEN B-66 and EMULGEN A-90 was demonstrated by comparing their performance against that of other surfactants using two different means: comparison of the kinetic response from pure human HDL and LDL in serum-based test fluids and accuracy comparisons with patient samples. The kinetics of the human LDL fluid at 75 mg/dL compared to the human HDL fluid at 75 mg/dL in Figures 1 through 4 show a much larger response for a non-selective surfactant EMULGEN 109P (a polyoxyethylene lauryl ether produced by KAO Corp.,) than the response for the HDL selective surfactant EMULGEN B-66. Similar non-selectivity or partial selectivity was observed with the other surfactants and is summarized in Table 2.

Dry analytical elements, or dry slides, and their use are described in numerous publications, including U.S. Patent Nos. 4,123,528; 4,786,605; 3,992,158; 4,258,001; 4,670,381; and European Patent Application Nos. 051 183; 066 648. The layers of the element of the present invention can be self-supporting, but preferably, these layers are disposed on a suitable dimensionally stable, chemically inert support. A support choice should be compatible with the intended mode of detection. Useful support material include but are not limited to paper, metal, foils, polystyrenes, polyesters, polycarbonates, and cellulose esters.

In the direct HDLC dry slide, the level of HDLC selectivity achieved merely using the superior HDL selective surfactants was not sufficient to achieve the accuracy targets for a single step assay. It was found that in order to achieve further improvement in HDLC selectivity, it was desirable to couple the HDL selective surfactant with phosphotungstic acid (PTA) precipitation. PTA, a classical method for achieving HDLC selectivity, facilitates HDLC selectivity by precipitating non-HDL. With the addition of PTA, both EMULGEN B-66 and EMULGEN A-90 showed similar HDLC selectivity while the other surfactants showed little or no HDLC selectivity. (Table 4) For example in Table 4, the HDLC selectivity of EMULGEN A-60 (a polyoxyethylene derivative produced by KAO Corp.,) and EMULGEN 109P with PTA is similar to that of non-specific surfactant TX-100, suggesting that both EMULGEN A-60 and EMULGEN 109P have no HDLC selectivity. In contrast, EMULGEN 220 (a polyoxyethylene cetyl ether produced by KAO Corp.,) shows intermediate HDLC selectivity in the presence of PTA.

An additional unique feature of EMULGEN B-66 and EMULGEN A-90 is their compatibility with PTA precipitated non-HDL. Both surfactants do not resolubilize the PTA-MgCl₂-non-HDL complexes. Although it has been documented that a surfactant's HLB number in solution methods is a good indicator of the its ability to solubilize certain proteins, in a direct HDLC dry slide the HLB number is a poor indicator. It has been shown that in the direct HDLC dry slide there is no correlation between the surfactant's HLB number (Table 1) and its ability to selectively solubilize HDL while not disrupting the PTA-MgCl₂-non-HDL complexes (correlation coefficient = 0.017). In contrast, EMULGEN B-66 and EMULGEN A-90's inherent HDL selectivity and the lack of solubilization of PTA precipitated non-HDL complexes are the essential properties responsible for the possibility of a single-step direct HDLC dry slide assay.

A "sample" as used herein, refers to any substance that may contain the analyte of interest. A sample can be biological fluid, such as whole blood or whole blood components including red blood cells, white blood cells, platelets, serum and plasma, ascites, urine, cerebrospinal fluid, and other constituents of the body which may contain the analyte of interest. Optionally, samples may be obtained from water, soil, and vegetation.

In at least one of the layers of the element of this invention is a dye which is capable of reacting with an enzyme to form a color. The dye functions as an indicator of the presence and the amount of HDLC present in a given sample. In a preferred embodiment of this invention, a leuco dye is used that can react with hydrogen peroxide and peroxidase to form a color. The color can be detected optically by the naked eye, by a photodiode selected to respond to a particular wavelength of light, or by other optical detection systems known by those skilled in the art using absorption, reflectance, or fluorescence spectroscopy. In a preferred embodiment of this invention, a reflectometer is used to detect and quantitate the dye color.

The element of this invention can include a wide variety of additives in appropriate layers as are known in the art to aid in manufacture, fluid spreading, and absorption and unwanted radiation.

The element of the present invention can be prepared using conventional coating procedures and equipment as are described in the art including gravure, curtain, hopper, and other coating techniques. The element can be configured in a variety of forms, including elongated tapes of any desired width, sheets, slides or chips. The process can be manual or automated.

The following examples are intended to illustrate, not limit the scope of the present invention.

### Example 1

Several surfactants were evaluated in a dry slide in order to evaluate their HDL selectivity. Below is an example of a multilayer analytical element, or dry slide, used in the evaluative process.

| MgCl₂/Surfactant |
|---|
| BaSO₄ Spreadlayer/Surfactant |
| I-100 Adhesion |
| Gel/COD/CEH |
| Gel/Dye/POD |

### Example 2

Table 1 details the surfactants screened in the direct HDLC dry slide.

**Table 1**

| Vendor | Surfactant | HLB Number |
|---|---|---|
| Kao Corp | EMULGEN A-60 | 12.8 |
| Kao Corp | EMULGEN B-66 | 13.2 |
| Kao Corp | EMULGEN A-90 | 14.5 |
| Kao Corp | EMULGEN 109P | 13.6 |
| Kao Corp | EMULGEN 220 | 14.2 |
| Dow Chemicals | TRITON X-00 | 13.5 |

### Example 3

Serum based test fluids containing pure human HDL and LDL were reacted with HDL specific EMULGEN B-66 surfactant and non-HDL specific EMULGEN 109P surfactant in a dry slide assay. The kinetic response for each reaction was recorded and shown in Figures 1 through 4.

### Example 4

The HDL selectivity of each surfactant in Table 1 was screened by measuring and comparing each surfactant's kinetic response to serum based test fluids containing pure human HDL and LDL. The results were then normalized to EMULGEN B-66 with a lower normalized number signifying decreased HDLC selectivity.

As shown in Table 2, surfactants, EMULGEN B-66 and EMULGEN A-90, screened in the HDLC dry slide have less LDL reactivity compared to HDL reactivity than other screened surfactants. Furthermore, when evaluated with human HDL and LDL test fluids, EMULGEN B-66 and EMULGEN A-90 are shown to be considerably more selective for HDL than LDL. In addition, these surfactants have unique specificity characteristics not seen with other surfactants used in the well-known cholesterol enzymatic cascade.

**Table 2**

| Surfactant | LDL(75)/HDL(75) | Normalized HDLC Selectivity |
|---|---|---|
| EMULGEN A-60 | 0.881 | 0.36 |
| EMULGEN B-66 | 0.321 | 1.00 |
| EMULGEN A-90 | 0.365 | 0.88 |
| EMULGEN 109P | 0.976 | 0.33 |
| EMULGEN 220 | 0.991 | 0.32 |

### Example 5

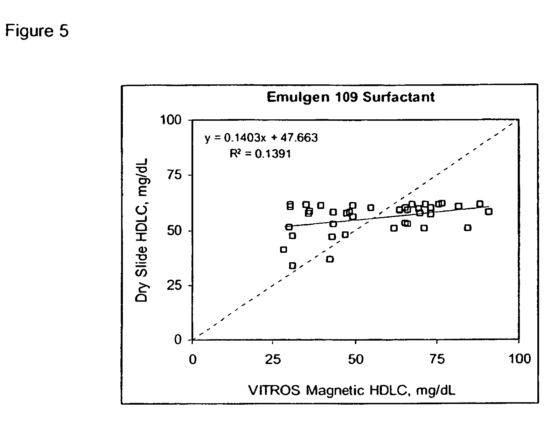

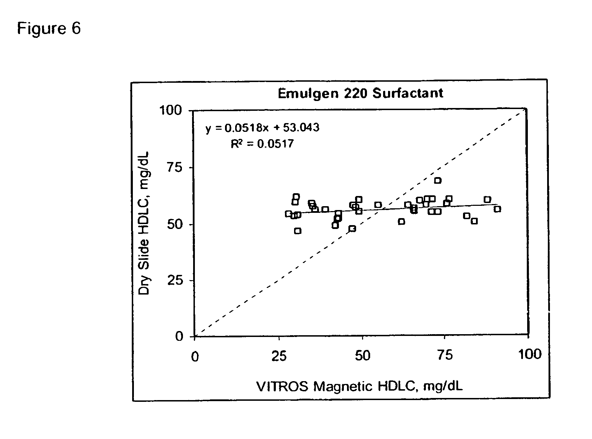

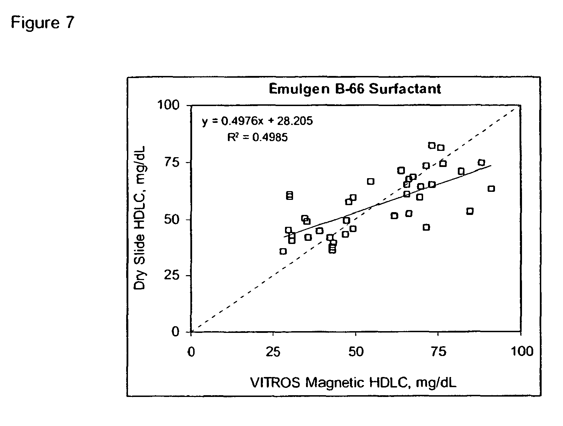

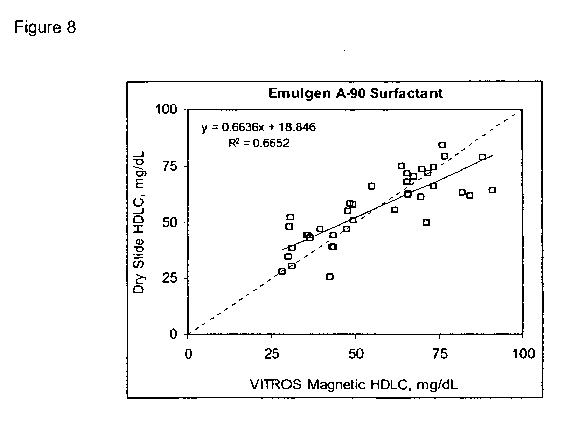

Further tests of HDLC selectivity were done using a split sample comparison with serum patient samples with varying concentrations of HDLC, also known as patient accuracy testing. The HDL selectivity of EMULGEN 109P, EMULGEN 220, EMULGEN B-66, and EMULGEN A-90 in direct HDLC dry slide assays were evaluated. As shown in Figures 5 through 8, the results from the dry slide assays where then compared to those obtained through the *VITROS* Magnetic HDLC precipitation method. The results from the direct HDLC slide assay were then correlated with the results from the *VITROS* Magnetic HDLC precipitation method and recorded in Table 3. The correlation results for the surfactants confirm the conclusions reached from the human HDL and LDL test fluids that EMULGEN B-66 and EMULGEN A-90 show HDL specificity that is not observed with other surfactants.

**Table 3**

| Surfactant Source | Slope | Intercept | Sy.x | R² |
|---|---|---|---|---|
| EMULGEN A-60 | 0.14 | 47.96 | 6.55 | 0.14 |
| EMULGEN E-66 | 0.50 | 28.21 | 9.40 | 0.50 |
| EMULGEN A-90 | 0.66 | 18.85 | 8.87 | 0.67 |
| EMULGEN 109P | 0.14 | 47.66 | 6.57 | 0.14 |
| EMULGEN 220 | 0.05 | 53.04 | 4.18 | 0.05 |

### Example 6

To test the effect of PTA on each surfactant's HDLC selectivity in a direct HDLC dry slide, PTA was added to the MgCl₂ and surfactant layer of the slide. Serum based test fluids containing pure human HDL and LDL were than used to test the HDLC selectivity of the surfactants. Each surfactant's HDLC selectivity resulting from these PTA added coatings are found in Table 4. TX-100, a general surfactant known to completely dissociate lipoproteins, was added as a control. The HDL selectivity results were then normalized to the EMULGEN B-66 data. (24). A lower normalized number signifies decreased selectivity to HDLC.

**Table 4**

| Surfactant | LDL(75)/HDL(75) | Normalized HDLC Selectivity | HDLC Selectivity +PTA/-PTA |
|---|---|---|---|
| EMULGEN A-60 | 0.941 | 0.32 | 1.07 |
| EMULGEN B-66 | 0.297 | 1.00 | 0.92 |
| EMULGEN A-90 | 0.222 | 1.34 | 0.61 |
| EMULGEN 109P | 0.992 | 0.30 | 1.02 |
| EMULGEN 220 | 0.621 | 0.48 | 0.63 |
| Triton X-100 | 0.994 | 0.30 | ND |

The formulas containing EMULGEN B-66 and EMULGEN A-90 showed the same HDLC selectivity with PTA while the other surfactants tested showed little or no HDLC selectivity with addition of PTA.

### Example 7

Table 4 also compares each surfactant's HDLC selectivity with and without the PTA coating using the human HDL and LDL linearity series. The dry slides containing EMULGEN B-66, EMULGEN A-90, and EMULGEN 220 all showed some enhancement of selectivity with the addition of PTA. In contrast, EMULGEN A-60 and EMULGEN 109P showed no enhancement of selectivity with the addition of PTA.

### Example 8

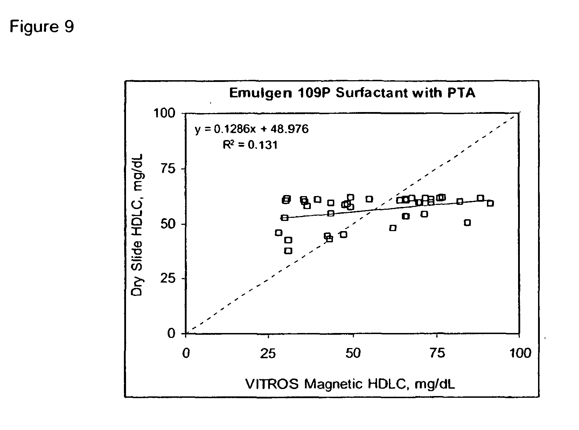

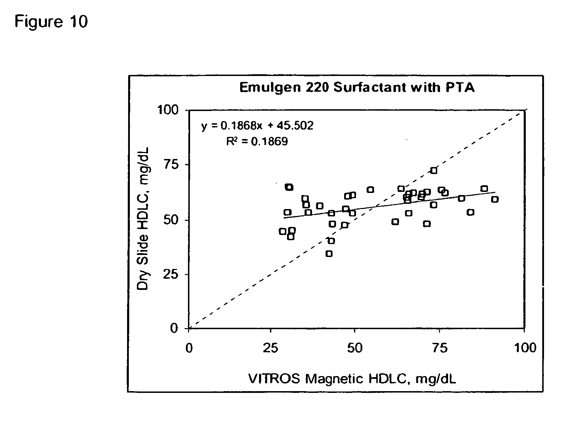

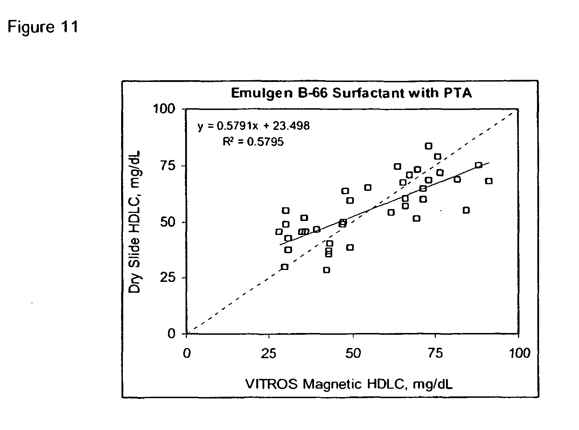

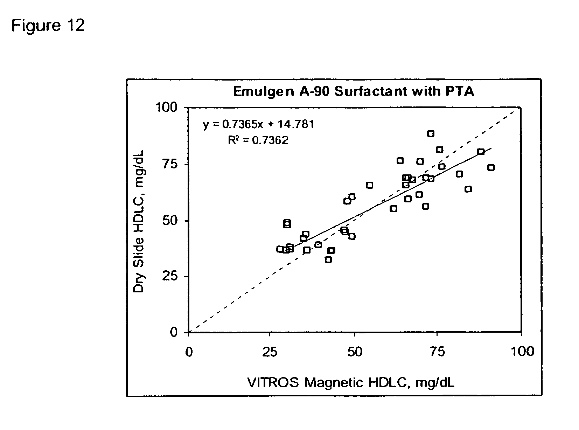

A similar comparison to the one in Example 7, comparing each surfactant's HDLC selectivity with and without the PTA coating was also performed using patient samples. Patient accuracy plots comparing the use of non-selective surfactants, EMULGEN 109P and EMULGEN 220, with the HDL selective surfactants, EMULGEN B-66 and EMULGEN A-90, in the dry slide with the PTA addition versus the *VITROS* Magnetic HDLC precipitation method (Figures 9-12). Table 5 depicts the correlation results between the direct HDLC slide and the *VITROS* Magnetic HDLC precipitation method for coatings containing PTA. Both EMULGEN B-66 and EMULGEN A-90 show HDLC selectivity while the other surfactants show little or no HDLC selectivity. The results using patient samples is also similar to those found with human HDL and LDL test fluids (Example 7) because EMULGEN B-66, EMULGEN A-90, and EMULGEN 220 all showed some enhancement of HDLC selectivity with the addition of PTA.

**Table 5**

| Surfactant Source | Slope | Intercept | Sy.x | r² |
|---|---|---|---|---|
| EMULGEN A-60 | 0.08 | 50.91 | 4.98 | 0.08 |
| EMULGEN B-66 | 0.58 | 23.50 | 9.29 | 0.58 |
| EMULGEN A-90 | 0.74 | 14.78 | 8.30 | 0.74 |
| EMULGEN 109P | 0.13 | 48.98 | 6.24 | 0.13 |
| EMULGEN 220 | 0.19 | 45.50 | 7.34 | 0.19 |
| Triton X-100 | 0.11 | 50.13 | 5.83 | 0.11 |

### Example 9

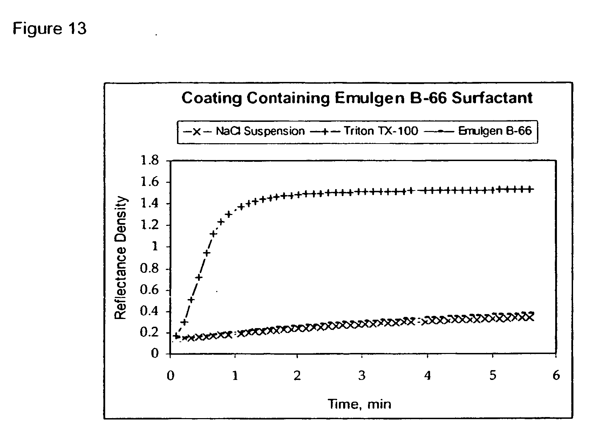

To demonstrate that these surfactants selectively solubilize HDL while leaving the complexes of non-HDL lipoprotein with PTA and MgCl₂ intact in solution as well as in the dry slide assay, classical techniques where used to precipitate and isolate the PTA-MgCl₂-non HDL precipitated complex (4). The precipitate was collected by centrifugation and washed, then resuspended in NaCl, 1% EMULGEN B-66, or 1% TX-100. The NaCl treatment remained a cloudy suspension while the TX-100 treatment cleared completely. However, the EMULGEN B-66 solution cleared slightly, but remained predominantly cloudy. These three treatments were analyzed on a coating similar to the PTA/EMULGEN B-66 coating described above. The kinetics are shown in Figure 13. The kinetics of the TX-100-treated non-HDL precipitate are very rapid, confirming that the LDL has been resolubilized prior to application to the slide. Both the EMULGEN B-66 and NaCl-treated samples show much slower, but very similar kinetics. This confirms that the non-HDL has remained precipitated and is only slowly resolubilized. The kinetics of the EMULGEN B-66 sample are slightly faster than the NaCl sample, confirming the hypothesis that very little resolubilization of non-HDL precipitate occurs in the presence of the EMULGEN B-66 surfactant in solution or in the direct HDLC dry slide.

### Example 10

In a preferred embodiment of the invention, shown below, the surfactant used in the dry slide format is either HDL selective surfactant, EMULGEN B-66 or EMULGEN A-90.

| |
|---|
| PTA/MgCl₂ |
| BaSO₄ Spreadlayer/Surfactant |
| I-100 Adhesion |
| PEG/COD/CEH |
| Gel/Dye |
| Gel/Dye/POD |

Table 6 shows the correlation results between patient samples on the direct HDLC dry slide in the preferred embodiment and the *VITROS* Magnetic HDLC precipitation method. Results of patient accuracy tests (Table 6) indicate that EMULGEN B-66 confers more selectivity to the formula than EMULGEN A-90 in the preferred embodiment shown above. The selectivity observed for both EMULGEN B-66 and EMULGEN A-90 is better in this format than demonstrated previously (Figures 9 through 12 and Table 5) due to the enhancements in the slide structure and optimization of the active reagents.

**Table 6**

| Enzyme Source | Slope | Intercepts | Sy.x | R² |
|---|---|---|---|---|
| EMULGEN B-66 | 0.82 | 11.03 | 7.11 | 0.78 |
| EMULGEN A-90 | 0.76 | 14.10 | 5.83 | 0.82 |
| EMULGEN B-66* | 0.89 | 6.53 | 4.89 | 0.90 |
| EMULGEN A-90* | 0.81 | 11.20 | 4.87 | 0.88 |

| | | | | |
|---|---|---|---|---|
| *One discrepant patient sample excluded from the data. | | | | |

### Example 11

By incorporation of the above discoveries in the dry slide, an accurate, precise, and rapid HDLC assay has been developed. In another preferred embodiment of the invention, shown below, the surfactant used in the dry slide format is the HDL selective surfactant EMULGEN B-66.

| |
|---|
| PTA/MgCl₂ |
| BaSO₄ Spreadlayer/Surfactant |
| I-100 Adhesion |
| PEG/COD/CEH |
| PEG/Dye |
| Gel/POD |

Direct HDLC dry slides were made using the formula and format described as the preferred slide structure above, using 7 g/m² EMULGEN B-66 as the HDL selective surfactant, *Candida rugosa* lipase or Denka CEH as the HDL selective cholesterol ester hydrolase, and IMnAg or PEG as the -02 matrix. Accuracy versus the *VITROS* Magnetic HDLC precipitation method and pooled precision were evaluated using 30 patient samples. Results can found in Table 7. The results show that this assay has acceptable accuracy and precision and is free from significant interference from hemolysed patient samples.

**TABLE 7 - Precision, Accuracy, and Hemolysate Interference Results from a Dry Analytical Element Assay for HDLC Using the Preferred Format.**

| -02 Matrix | CEH Source | Patient Accuracy | | | Precision | Interference |
|---|---|---|---|---|---|---|
| | | Slope | Intercept | Sy.x | Pooled SD | Hemolysate |
| PEG | Denka | 0.99 | 0.50 | 2.2 | 1.30 | >500 mg/dL Hb |
| IMnAg | *Candida rugosa* | 0.99 | 0.52 | 2.46 | 3.89 | >500 mg/dL Hb |

### Literature Cited

1 Burstein, M., Scholnick, H.R., & Morfin, R. J Lipid Res, 1970, 11, 583-595.
2 Fredrickson, D.S., Levy, R.I., & Lindgren, F.T. J Clin Invest 1968, 47, 2446-2457.
3 Warnick, G., Benderson, J., & Albers, J.J. Clin Chem 1979, 25,1309-1313.
4 Burstein, M., & Scholnick, H.R. Adv Lipid Res 1973, 11, 67-108.
5 Briggs, C., Anderson, D., Johnson, P., & Deegan, T. Ann Clin Biochem 1981, 18, 177-181.
6 Kakuyama, T., Kimura, S., & Hasiguchi, Y. Clin Chem 1994, 40, A1104.
7 Nauck, M., März, W., & Wieland, H. Clin Chem 1998, 44, 1443-1451.
8 Arranz-Pena, M., Tasende-Mata, J., & Martin-Gil, F. J. Clin Chem 1998, 44, 2499-2505.
9 Yamamoto, M., Nakamura, M., Hino, K., Saito, K., & Manabe, M. Clin Chem 2000, 46, A98
10 Matsui, H., Ito, Y., Ohara, S., & Fujiwara, A. *European Patent* EP 0887422A1, Priority Date 09/12/1996.
11 Hino, K., Nakamura, M., & Manabe, M. *United States Patent* 5,773,304, Priority Date 1/31/1995.
12 Izawa, S., Okada, M., Matsui, H., & Horita, Y. J Med Pharm Sci 1997, 37, 1385-1388.
13 Sugiuchi, H., Uji, Y., Okabe, H., Irie, T., Uekama, K., Kayahara, N., & Miyauchi, K. Clin Chem 1995, 41, 717-723.
14 Neugebauer, J. M. Methods Enzymol. 1990, 182, 239-253.
15 Hjelmeland, L. M. Methods Enzymol. 1990, 182, 253-264.
16 Marston, F. A. O., & Hartley, D. L. Methods Enzymol, 1990, 182, 264-276.
17 Hjelmeland, L. M. Methods Enzymol. 1990, 182, 277-282.
18 Egan, R. W. J Biol Chem 1976, 251, 4442-4447.
19 Slinde, E., & Flatmark, T. Biochim Biophys Acta 1976, 455, 796-805.
20 Tucker, I. G., & Florence, A. T. J Pharm Pharmocol 1983, 35, 705-711.
21 Kishi, K., Ochiai, K., Ohta, Y., Uemura, Y., Kanatani, K., Nakajima, K., Wang, T., & Nakamura, M. 69th AACC 2001, Poster, Chicago, IL.
22 Lawlor, J.F., & Musto, J.D. United State Patent 5,242,833, September 7, 1993.

## Claims

1. A method for quantifying cholesterol in high-density lipoprotein in a single step assay, comprising:
(a) Providing a multi-layer analytical element wherein at least one layer contains phosphotungstic acid and at least one layer contains a surfactant that selectively acts on high-density lipoproteins and does not solubilize non-high density lipoprotein precipitated complexes,
(b) Contacting the multi-layer analytical element with a sample that may contain high-density lipoprotein cholesterol, and
(c) Detecting and quantifying the high-density lipoprotein cholesterol.

2. The method claimed according to claim 1, wherein the surfactant is selected from the group consisting of EMULGEN B-66, EMULGEN A-90, and a mixture thereof.

3. The method claimed according to claim 1, wherein phosphotungstic acid is present in the amount of 1 to 5 g/m².

4. The method claimed according to claim 1, wherein surfactant is present in the amount of 3 to 8 g/m².

## Patentansprüche

1. Verfahren zur Quantifizierung von Cholesterin in High-Density-Lipoprotein in einem Ein-Schritt-Test, wobei man:
(a) ein mehrschichtiges Analyseelement bereitstellt, wobei wenigstens eine Schicht Wolframatophosphorsäure enthält und wenigstens eine Schicht ein Tensid enthält, das selektiv auf High-Density-Lipoproteine einwirkt und präzipitierte Nicht-High-Density-Lipoprotein-Komplexe nicht solubilisiert,
(b) das mehrschichtige Analyseelement mit einer Probe in Kontakt bringt, die möglicherweise High-Density-Lipoprotein-Cholesterin enthält, und
(c) das High-Density-Lipoprotein-Cholesterin nachweist und quantifiziert.

2. Verfahren nach Anspruch 1, wobei das Tensid aus der aus EMULGEN B-66, EMULGEN A-90 und einem Gemisch davon bestehenden Gruppe ausgewählt ist.

3. Verfahren nach Anspruch 1, wobei Wolframatophosphorsäure in der Menge von 1 bis 5 g/m² vorliegt.

4. Verfahren nach Anspruch 1, wobei Tensid in der Menge von 3 bis 8 g/m² vorliegt.

## Revendications

1. Méthode de quantification du cholestérol dans une lipoprotéine de haute densité dans un dosage en une seule étape, comprenant :
(a) Le fait de se munir d'un élément analytique multicouche où au moins une couche contient de l'acide phosphotungstique et au moins une couche contient un tensioactif qui agit sélectivement sur les lipoprotéines de haute densité et ne solubilise pas les complexes précipités de lipoprotéine non de haute densité,
(b) La mise en contact de l'élément analytique multicouche avec un échantillon qui peut contenir du cholestérol de lipoprotéine de haute densité, et
(c) La détection et la quantification du cholestérol de lipoprotéine de haute densité.

2. Méthode selon la revendication 1, où le tensioactif est choisi dans le groupe constitué par EMULGEN B-66, EMULGEN A-90, et leurs mélanges.

3. Méthode selon la revendication 1, où l'acide phosphotungstique est présent à une teneur comprise entre 1 et 5 g/m².

4. Méthode selon la revendication 1, où le tensioactif est présent à une teneur comprise entre 3 et 8 g/m².
